# EUROPEAN PATENT APPLICATION

(11) **EP 2 253 627 A1**
(43) Date of publication of application: **24.11.2010**
(21) Application number: 09425196.4
(22) Date of filing: 21.05.2009
(51) Int. Cl.: C07D 333/56, A61P 19/00, A61K 31/381

(54) **Inclusion complex of raloxifene hydrochloride and beta-cyclodextrin.**

(71) Applicant: ERREGIERRE S.p.A., 24060 San Paolo D'Argon (Bergamo) (IT)
(72) Inventor: Ferrari, Massimo, 24069 Cenate Sotto (IT); Gargani, Pietro Carlo, 20141 Milano (IT)
(74) Representative: Cattaneo, Elisabetta

(57) **Abstract**

There is described an inclusion complex of raloxifene hydrochloride and β-cyclodextrin in the solid phase having a specific X-ray powder diffraction spectrum. The invention also relates to a process for obtaining the raloxifene hydrochloride inclusion complex with β-cyclodextrin of the invention. The complex of the invention is employed as a pharmaceutical ingredient in the treatment of osteoporosis, specifically postmenopausal osteoporosis.

## Description

### FIELD OF THE INVENTION

The present invention relates to a inclusion complex of raloxifene hydrochloride and β-cyclodextrin and to its use in the medical field.

### STATE OF THE ART

Raloxifene hydrochloride is an active compound employed in the treatment of osteoporosis, specifically of postmenopausal osteoporosis. It has the following formula:

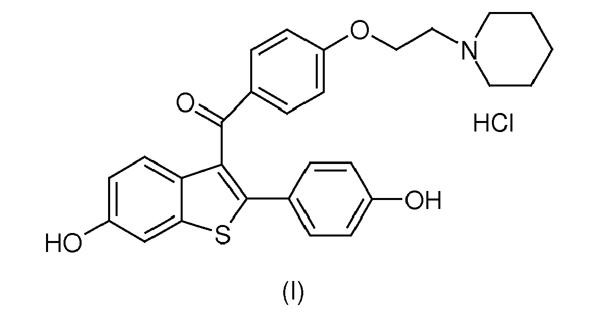

In spite of the evident therapeutic interest, the raloxifene hydrochloride salt has the drawback of being poorly water soluble at ambient temperature. Such a poor solubility leads to the fact that it should be administered as a water suspension, with the aid of suitable suspending agents, reducing thus the administration possibilities thereof.

Indeed, a water soluble formulation is desirable, as it provides a better dispersion of the active ingredient and a simultaneous better safety of administration.

US Patent No. 5,624,940 describes an aqueous solution composition of an inclusion complex formed by a water soluble cyclodextrin and compounds of Formula I, wherein raloxifene may be identified. Therefore, in the US document, aqueous solutions of inclusion complexes of poorly water soluble active ingredients with hydroxypropyl- β-cyclodextrin are prepared and pharmaceutical formulations of these complexes are described, which have blood plasma levels 15 times higher than those having the same dosage and prepared as a wet granular formulation.

US application US2006/0105045 describes a composition comprising a water miscible organic solvent, a cyclodextrin derivative and a compound and a method to enhance the compound solubility in an aqueous solution comprising the formation of complexes between such a compound and the cyclodextrin in an aqueous medium. Raloxifene is included in the long list of active ingredients which are poorly water soluble and suitable to form inclusion complexes with hydroxy-butenyl-β-cyclodextrin in case of an organic solvent is present.

At present, there is a need to provide a solid form of raloxifene, which is advantageously suitable for preparing and processing in pharmaceutical field, while having a good stability during the processing and solubility when taken to a liquid phase for common usage.

The object of the present invention is thus to provide a raloxifene compound which is in the solid phase.

### SUMMARY

Therefore, the above-mentioned object has been achieved by providing an inclusion complex of raloxifene hydrochloride and β-cyclodextrin in the solid phase.

The invention further concerns a process for obtaining the inclusion complex of the invention comprising the following steps:
A) dissolving raloxifene hydrochloride in an aqueous solution;
B) heating the mass to a temperature ranging from 50 to 80°C;
C) adding β-cyclodextrin;
D) separating the raloxifene hydrochloride-β-cyclodextrin complex by precipitation and filtration.

### DESCRIPTION OF THE DRAWINGS

The features and advantages of the invention will become apparent from the following detailed description and accompanying drawings, in which:
- Figure 1 shows the X-ray diffractogram of the inclusion complex of the invention;
- Figures 2a, 2b, 2c show the comparison of the experimental diffractograms of the complex of the invention (2a), the raloxifene hydrochloride (2b) and the β-cyclodextrin (2c);
- Figures 3a, 3b, 3c show the comparison of the infrared spectra of the complex of the invention (3a), the raloxifene hydrochloride (3b) and β-cyclodextrin (3c).

### DETAILED DESCRIPTION OF THE INVENTION

Therefore, the invention relates to a raloxifene hydrochloride and β-cyclodextrin inclusion complex.

The inclusion complex of the invention has been characterized by X-ray diffraction, which confirmed the actual formation of the complex, since the obtained peaks were not the result of the sum of the peaks related to the two separate components.

The raloxifene hydrochloride-β-cyclodextrin inclusion complex has the following peaks at diffraction degrees (2-theta) in the X-ray powder diffraction spectrum ±0.2:
10.6, 12.4, 14.4, 15.3, 19.0, 19.5.

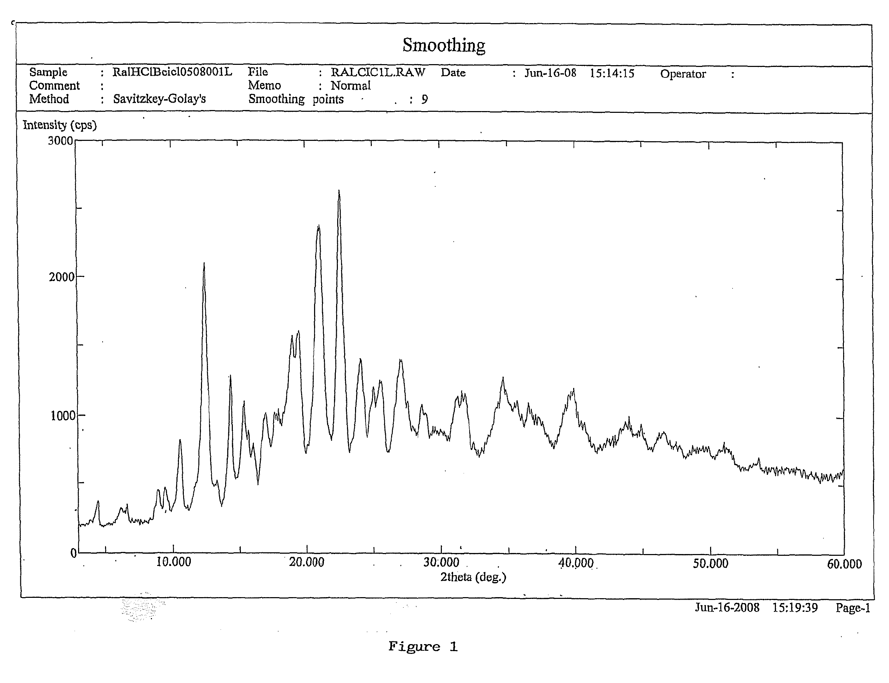

Specifically, the inclusion complex of the invention has 57 peaks at the diffraction degrees and relative intensity shown in the following Table 1, according to the diffraction spectrum shown in Figure 1.

**Table 1: Peaks of the raloxifene HCl-β-cyclodextrin inclusion complex (2-theta in angular degrees ±0.2°)**

| Peak | 2-theta | Intensity (cps) | I/I₀ |
|---|---|---|---|
| 1 | 4.4 | 370 | 15 |
| 2 | 6.2 | 320 | 13 |
| 3 | 6.6 | 351 | 14 |
| 4 | 8.9 | 452 | 18 |
| 5 | 9.4 | 463 | 18 |
| 6 | 10.6 | 817 | 32 |
| 7 | 11.7 | 480 | 19 |
| 8 | 12.4 | 2086 | 80 |
| 9 | 13.3 | 524 | 21 |
| 10 | 14.4 | 1284 | 50 |
| 11 | 15.3 | 1095 | 42 |
| 12 | 15.7 | 891 | 35 |
| 13 | 16.0 | 798 | 31 |
| 14 | 17.0 | 1016 | 39 |
| 15 | 17.7 | 1017 | 39 |
| 16 | 18.0 | 1039 | 40 |
| 17 | 19.0 | 1571 | 61 |
| 18 | 19.5 | 1602 | 62 |
| 19 | 21.1 | 2364 | 91 |
| 20 | 22.6 | 2615 | 100 |
| 21 | 24.0 | 1405 | 54 |
| 22 | 24.8 | 1049 | 41 |
| 23 | 25.0 | 1195 | 46 |
| 24 | 25.6 | 1237 | 48 |
| 25 | 26.8 | 1276 | 49 |
| 26 | 27.1 | 1378 | 53 |
| 27 | 27.5 | 1095 | 42 |
| 28 | 28.6 | 1063 | 41 |
| 29 | 29.0 | 1009 | 39 |
| 30 | 30.0 | 900 | 35 |
| 31 | 30.3 | 874 | 34 |
| 32 | 31.2 | 1122 | 43 |
| 33 | 31.8 | 1146 | 44 |
| 34 | 32.0 | 981 | 38 |
| 35 | 32.5 | 807 | 31 |
| 36 | 33.1 | 766 | 30 |
| 37 | 33.5 | 851 | 33 |
| 38 | 34.6 | 1248 | 48 |
| 39 | 35.6 | 1104 | 43 |
| 40 | 36.0 | 1007 | 39 |
| 41 | 36.5 | 1090 | 42 |
| 42 | 37.3 | 984 | 38 |
| 43 | 37.6 | 946 | 37 |
| 44 | 39.5 | 1142 | 44 |
| 45 | 39.9 | 1193 | 46 |
| 46 | 40.7 | 944 | 37 |
| 47 | 41.4 | 798 | 31 |
| 48 | 42.4 | 817 | 32 |
| 49 | 43.3 | 911 | 35 |
| 50 | 43.7 | 971 | 38 |
| 51 | 44.9 | 946 | 37 |
| 52 | 45.2 | 844 | 33 |
| 53 | 46.6 | 887 | 34 |
| 54 | 47.5 | 794 | 31 |
| 55 | 47.8 | 795 | 31 |
| 56 | 51.5 | 727 | 28 |
| 57 | 53.6 | 704 | 27 |

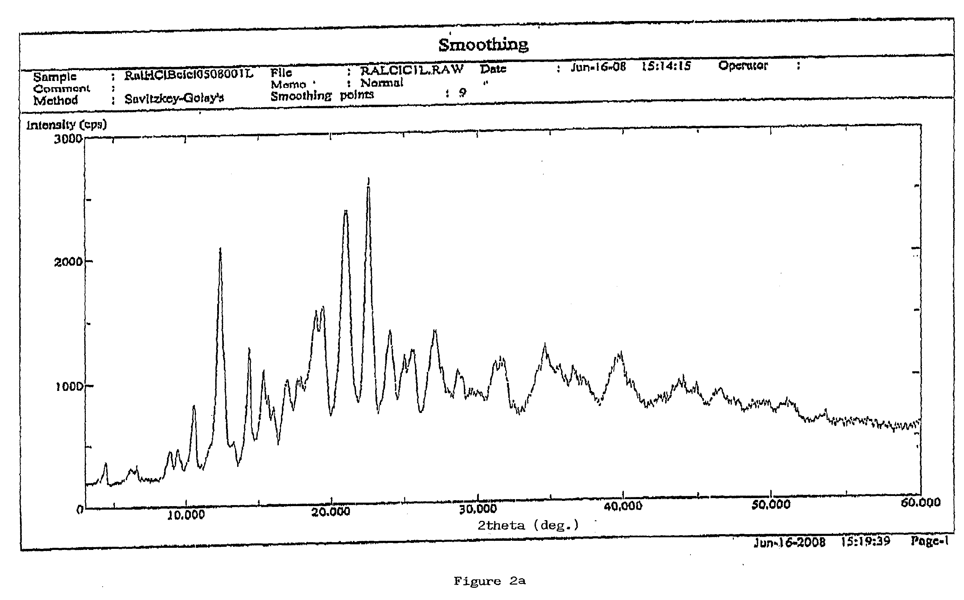

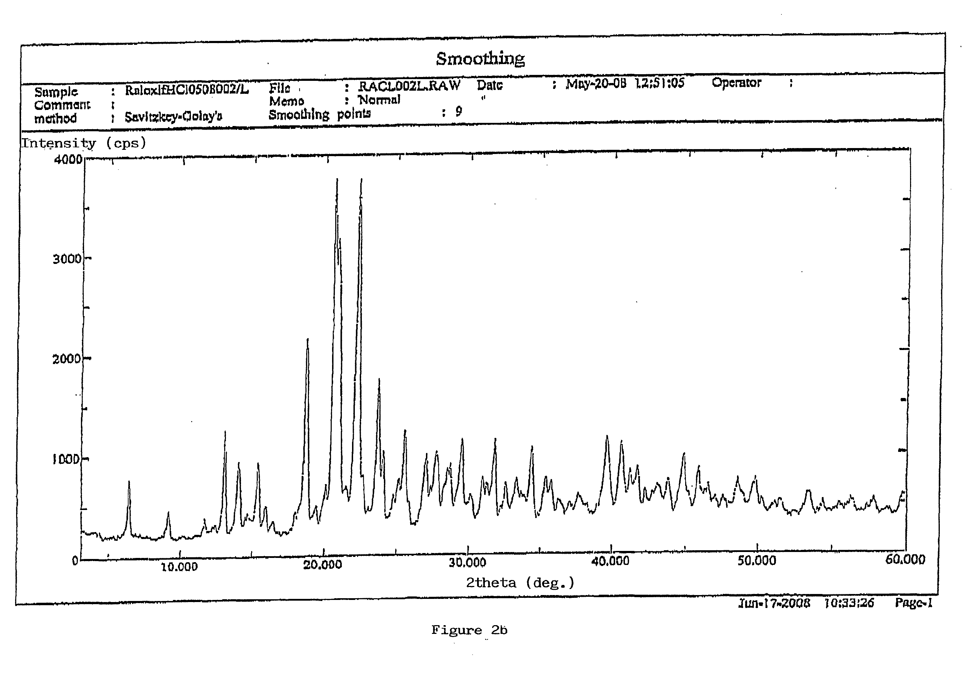

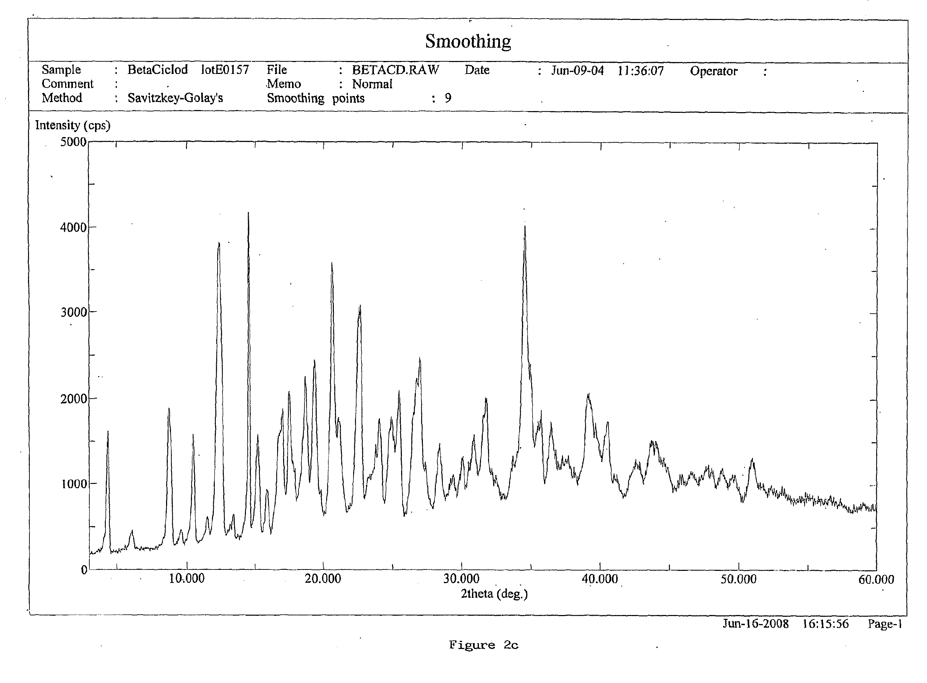

The comparison of the spectra of the complex (2a), the raloxifene hydrochloride (2b) and the β-cyclodextrin (2c) may be derived from Figures 2a, 2b, 2c. As it is evident from the Figures, the inventors of the present invention could indeed confirm that the inclusion complex corresponded to a new crystalline form right by means of the X-ray diffraction analysis. In fact, they performed the X-ray analysis of β-cyclodextrin and raloxifene hydrochloride and could underline that the peaks existing in the complex diffractogram did not result from the sum of peaks related to the two separate components, thus pointing out the successful inclusion of raloxifene hydrochloride into cyclodextrin.

The invention further concerns a process for obtaining the raloxifene hydrochloride inclusion complex with β-cyclodextrin comprising the following steps:
A) dissolving raloxifene hydrochloride in an aqueous solution;
B) heating the mass to a temperature ranging from 50 to 80°C;
C) adding β-cyclodextrin;
D) separating the raloxifene hydrochloride-β-cyclodextrin complex by precipitation and filtration.

Preferably, in step a) a water-methanol solution is used as the aqueous solution, whose weight/weight ratio is 2:1.

Preferably, the cyclodextrin is added in step c) at a ratio of 1:1 with respect to the raloxifene hydrochloride.

Therefore, as will be evident from the following experimental section, the inclusion complex of the invention has been further characterized by means of ¹³C-NMR nuclear magnetic resonance, ¹H-NMR nuclear magnetic resonance, infrared spectroscopy (IR).

The product of the invention advantageously had a purity of 99.8%.

The product was tested for its water solubility, both as it is and as a micronized product, and it turned out to be particularly soluble as will be demonstrated in the following experimental section.

The inclusion complex turned out to be hygroscopic over time up to a maximum water content of 12%. Such a range of water content corresponded, however, to absorption water, which is removable by means of an oven treatment, and not to crystallization water.

The inclusion complex of the invention may be used as a medicament.

Therefore, it may be combined with a pharmaceutically acceptable vehicle, and optionally suitable excipients, to obtain pharmaceutical compositions. The term "pharmaceutically acceptable vehicle" is meant to include solvents, support agents, diluents and the like, which are all used in administering the inclusion complex of the invention.

These pharmaceutical compositions may be parenterally, orally or topically administered.

The compositions of the present invention suitable for oral administration will be conveniently in the form of discrete units such as tablets, capsules, cachets, as powders or granules, or further as a liquid suspension.

More preferably, the compositions of the invention for oral administration will be in the form of tablets.

The compositions for parenteral administration will conveniently comprise sterile preparations.

The compositions for topical administration will conveniently be in the form of creams, pastes, cataplasms, oils, ointments, emulsions, foams, gels, drops, aqueous solutions, spray solutions and transdermal patches.

The complex of the invention may be employed for manufacturing a medicament for the treatment of osteoporosis, specifically of postmenopausal osteoporosis.

The invention will be now described in further detail in the following examples, provided by way of non limitative illustration of the invention, related to the process of the invention and to the characterization of the inclusion complex obtained by the process.

### EXAMPLES

### Example 1

### Process for preparing the inclusion complex of raloxifene hydrochloride and β-cyclodextrin

20.0 g of raloxifene hydrochloride (0.0392 mol), 180 g of methanol and 360 g of water were mixed together. The mass was heated at 50-60°C until complete dissolution. 45.6 g of β-cyclodextrin (mol 0,040) were then added. The mass was heated to reflux until complete dissolution, 190 g of methanol-water mixture are then distilled under atmospheric pressure. The mass was cooled to 0-30°C and kept at such a temperature to assist the precipitation. The obtained product was then filtered by washing the panel with 40 g of water, and dried at 85-95°C. About 58 g of raloxifene hydrochloride - β-cyclodextrin were obtained.

The sample was subjected to elemental analysis and the results are shown in Table 2.

**Table 2**

| Element | % calculated for C₇₀H₉₈O₃₉NSCl · H₂O | % found |
|---|---|---|
| C | 50.55 | 50.37 |
| H | 6.02 | 6.15 |
| N | 0.84 | 0.88 |
| Cl | 2.14 | 2.18 |
| S | 1.96 | 2.02 |
| O | 38.52 | 38.05 |

### Example 2

### Characterization of the complex through X-Ray diffraction

The product obtained from example 1 was subjected to structural determination analysis.

Specifically, a sample was analyzed by means of a Rigaku Miniflex diffractometer and the radiations employed were α₁, and α₂ of copper (λ=1.54051Å; λ=1.54430 Å).

The analysis was repeated on a sample of raloxifene hydrochloride and a sample of β-cyclodextrin. The obtained spectra were shown in Figures 2b and 2c, respectively. It could be seen that the peaks existing in the complex did not result from the sum of peaks related to the two separate components, thus pointing out the successful inclusion of raloxifene hydrochloride into cyclodextrin with the consequent modification of the cell parameters.

The sample of the invention had the diffractogram shown in Figure 1, the peaks of which are indicated in Table 1 above.

### Example 3

### Characterization of the complex through ¹H-NMR Nuclear Magnetic Resonance

A sample of inclusion complex as obtained from example 1 was subjected to ¹H-NMR nuclear magnetic resonance analysis, employing a 200 MHz Varian Gemini as the instrument and DMSO-d₆ and DMSO-d₆ +D₂O as the solvent.

The obtained results were

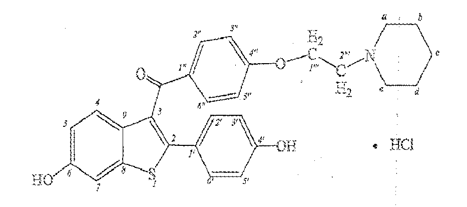

| δ ***(ppm)*** | *Multiplicity* | ***(H)*** | ***J(Hz)*** | *Assignation* |
|---|---|---|---|---|
| 1.35-1.85 | multiplets | (6) | n.m. | CH₂ (b), CH₂ (c), CH₂ (d), |
| 2.93 | multiplet | (2) | n.m. | CH₂(2"'), |
| 3.10-3.80 | overlapping systems* | 34 | n.m. | CH₂ (a), CH₂ (e) + [CH (2), CH (3), CH (4), CH (5), CH (6) *of the* β-*cyclodextrin*] |
| 4.30-4.50 | singulets | (8) | | CH₂ (1"'), + 6(OH) *of the* β*-cyclodextrin* |
| 4.81 | duplet | (6) | 1.6 | CH (1) β*-cyclodextrin* |
| 5.60-5.80 | singulets | (12) | | β*-cyclodextrin OH (exchanging with D20)* |
| 6.66 | AA'XX' system. part XX' | (2) | 8.5 | CH (3'), CH₂ (5') |
| 6.84 | | (1) | 2.9 | CH(5) |
| 6.95 | AA'YY' system, | (2) | 8.8 part YY' | CH(3"), CH₂ (5") |
| 7.15 | AA' XX' systempart AA' | (2) | 8.5 | CH(2'), CH₂ (6') |
| 7.24 | duplet | (1) | 9 | CH (4) |
| 7.33 | duplet | (1) | 2 | CH(7) |
| 7.68 | AA'YY' system, part AA' | | 8.8 | CH(2"), CH(6") |
| 9.78 and 9.81 | singulets | (2) | | phenolic OH (exchanging with D₂O) |
| 9.90 | Broaded singulet | (1) | | N-H⁺ (exchanging with D₂O) |

| | | | | |
|---|---|---|---|---|
| *n.m* = *not measurable* * the hydrogens bounded to C₂, C₃, C₄, C₅, C₆ of the β-cyclodextrin falls into this area: | | | | |

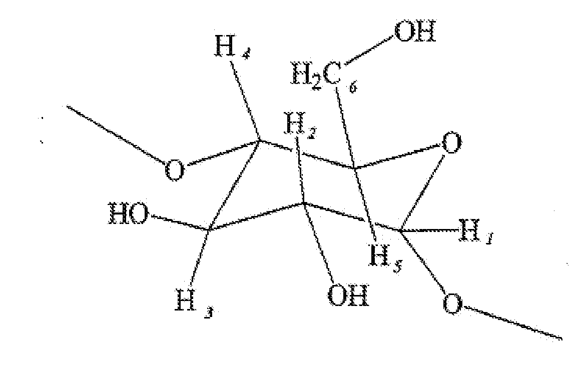

| δ***(ppm)*** | *Multiplicity* | ***(H)*** | *Assignation* |
|---|---|---|---|
| 3.29 | multiplet | (1) | CH(4) |
| 3.33 | multiplet | (1) | CH(2) |
| 3.58 | multiplet | (1) | CH(5) |
| 3.66 | multiplet | (1) | CH(3) |
| 3.63 | multiplet | (2) | CH₂(6) |
| 4.81 | duplet | (1) | CH(1) |

The results of the ¹H-NMR analysis pointed out a (1:1) intermolecular complexation between raloxifene hydrochloride and β-cyclodextrin.

### Example 4

### Characterization of the complex through ¹³C-NMR Nuclear Magnetic Resonance

A sample of inclusion complex as obtained from example 1 was subjected to ¹³C-NMR nuclear magnetic resonance analysis, employing a 50 MHz Varian Gemini as the instrument and DMSO-d₆ as the solvent.

The obtained results were

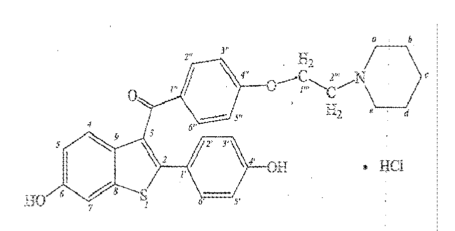

| δ***(ppm)*** | *Multiplicity* * | *Assignation* |
|---|---|---|
| 21.82 | *triplet* | CH₂ *(c)* |
| 23.03 | *triplet* | CH₂ *(b)*, CH₂ *(d)* |
| 53.33 | *triplet* | CH₂ *(a)*, CH₂ *(e)* |
| 55.26 | *triplet* | CH₂ *(2"')* |
| 60.61 | *triplet* | CH₂ *(6) of the* β*-cyclodextrin*** |
| 63.20 | *duplet* | CH₂ *(1"')* |
| 72.73 | *duplet* | CH₂ *(2) of the* β*-cyclodextrin* |
| 73.11 | *duplet* | CH₂ *(5) of the* β*-cyclodextrin* |
| 73.75 | *duplet* | CH₂ *(3) of the* β*-cyclodextrin* |
| 82.22 | *duplet* | CH₂ *(4) of the* β*-cyclodextrin* |
| 102.62 | *duplet* | CH₂ *(1) of the* β*-cyclodextrin* |
| 107.85 | *duplet* | CH (7) |
| 115.36 | *duplet* | CH *(3"),* CH *(5")* |
| 115.96 | *duplet* | CH *(5)* |
| 116.42 | *duplet* | CH *(3')* , CH *(5')* |
| 123.94 | *duplet* | CH (4) |
| 124.42 | *singulet* | C *(1')* |
| 130.22 | *singulet* | C*(2) or* C*(3) or* C*(1")* |
| 130.40 | *triplet* | CH*(2')* , CH *(6')* |
| 131.01 | *singulet* | C*(2) or* C*(3) or* C*(1")* |
| 132.51 | *duplet* | CH*(2") ,* CH *(6")* |
| 132.90 | *singulet* | C*(2) or* C*(3) or*C*(1")* |
| 139.49 | *singulet* | C*(8) or* C*(9)* |
| 141.28 | *singulet* | C*(9) or*C*(8)* |
| 156.17 | *singulet* | C*(6) or*C*(4')* |
| 158.57 | *singulet* | C*(4') or* C*(6)* |
| 162.38 | *singulet* | C*(4")* |
| 193.92 | *singulet* | C=O |

| | | |
|---|---|---|
| * = *without proton decoupling* ** The cyclodextrin I.C.S. were as follows: | | |

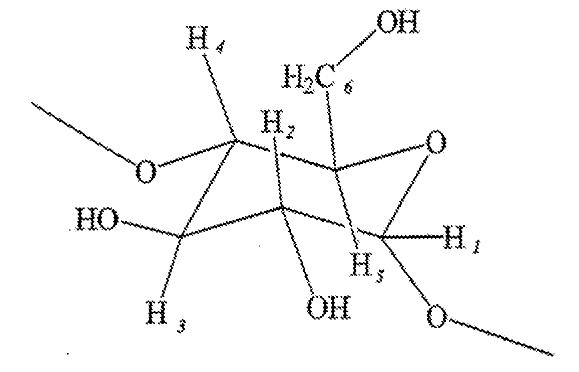

| δ***(ppm)*** | *Multiplicity* | *Assignation* |
|---|---|---|
| 60.620 | *triplet* | CH₂(6) |
| 72.728 | *duplet* | CH(2) |
| 73.107 | *duplet* | CH(5) |
| 73.736 | *duplet* | CH(3) |
| 82.237 | *duplet* | CH(4) |
| 102.634 | *duplet* | CH(1) |

The ¹³C-NMR spectrum was in accordance with the proposed structure.

### Example 5

### Characterization by means of infrared spectroscopy

A sample of the inclusion complex of example 1, suspended into KBr, was subjected to infrared spectroscopy by means of Perkin-Elmer Spectrum-one spectrophotometer.

44 peaks were obtained, which are quoted in cm⁻¹ in the following Table 3.

**Table 3: values of the infrared spectrum peaks of the inclusion complex of raloxifene hydrochloride-β-cyclodextrin**

| Peaks | cm⁻¹ |
|---|---|
| 1 | 3368.83 |
| 2 | 2943.95 |
| 3 | 2749.85 |
| 4 | 2693.58 |
| 5 | 2572.28 |
| 6 | 2541.62 |
| 7 | 2079.93 |
| 8 | 1915.62 |
| 9 | 1642.89 |
| 10 | 1610.98 |
| 11 | 1596.66 |
| 12 | 1581.56 |
| 13 | 1541.36 |
| 14 | 1499.75 |
| 15 | 1465.21 |
| 16 | 1430.34 |
| 17 | 1359.33 |
| 18 | 1338.19 |
| 19 | 1309.54 |
| 20 | 1259.24 |
| 21 | 1246.74 |
| 22 | 1235.46 |
| 23 | 1205.07 |
| 24 | 1158.05 |
| 25 | 1104.24 |
| 26 | 1079.34 |
| 27 | 1028.67 |
| 28 | 1001.58 |
| 29 | 948.49 |
| 30 | 938.14 |
| 31 | 923.26 |
| 32 | 906.13 |
| 33 | 888.34 |
| 34 | 839.63 |
| 35 | 807.03 |
| 36 | 782.52 |
| 37 | 760.80 |
| 38 | 723.87 |
| 39 | 705.91 |
| 40 | 642.92 |
| 41 | 622.42 |
| 42 | 609.49 |
| 43 | 585.13 |
| 44 | 529.29 |

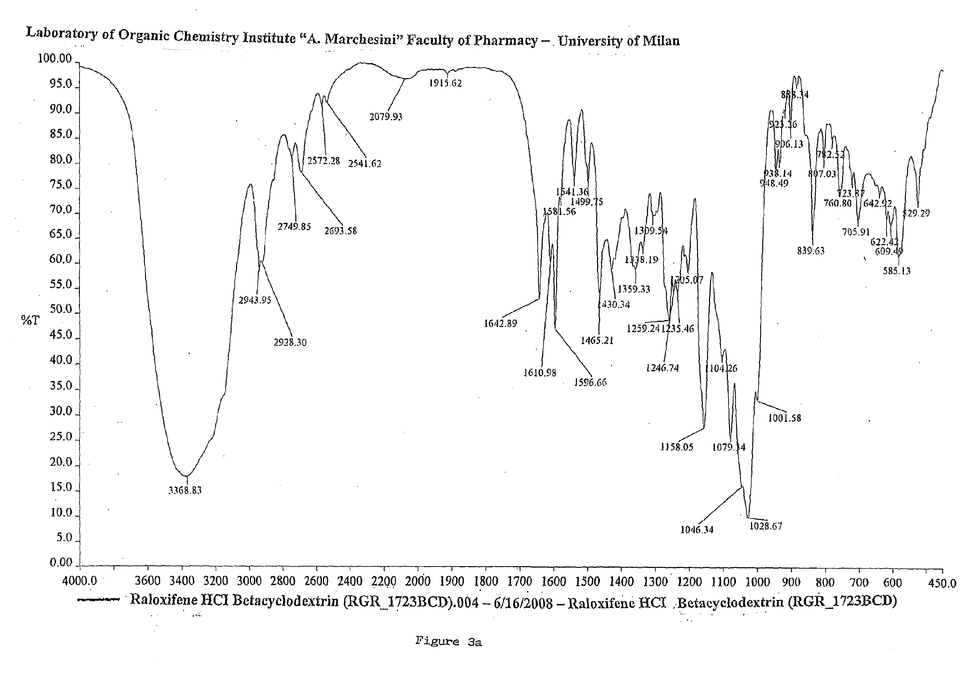

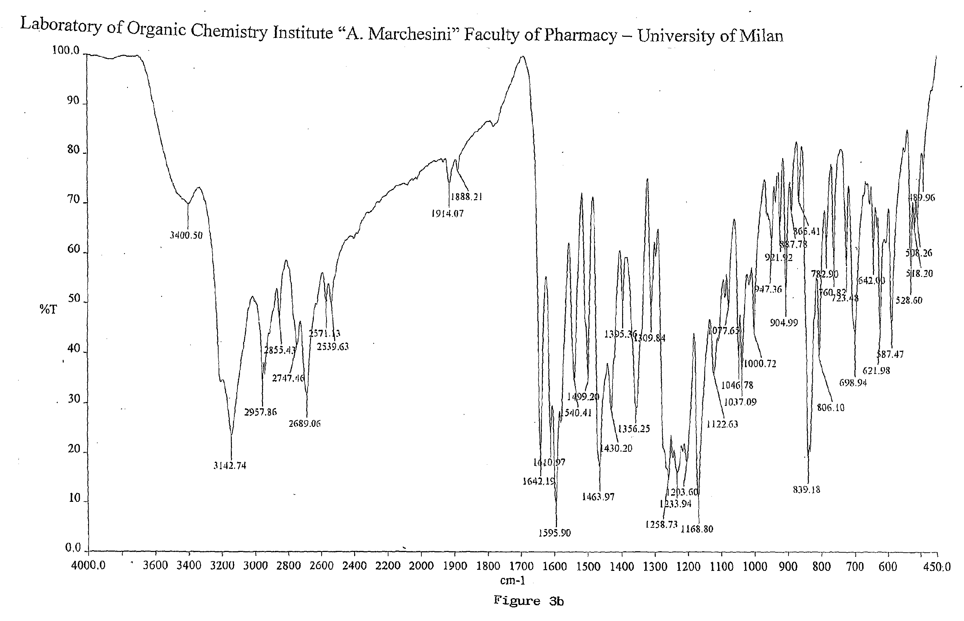

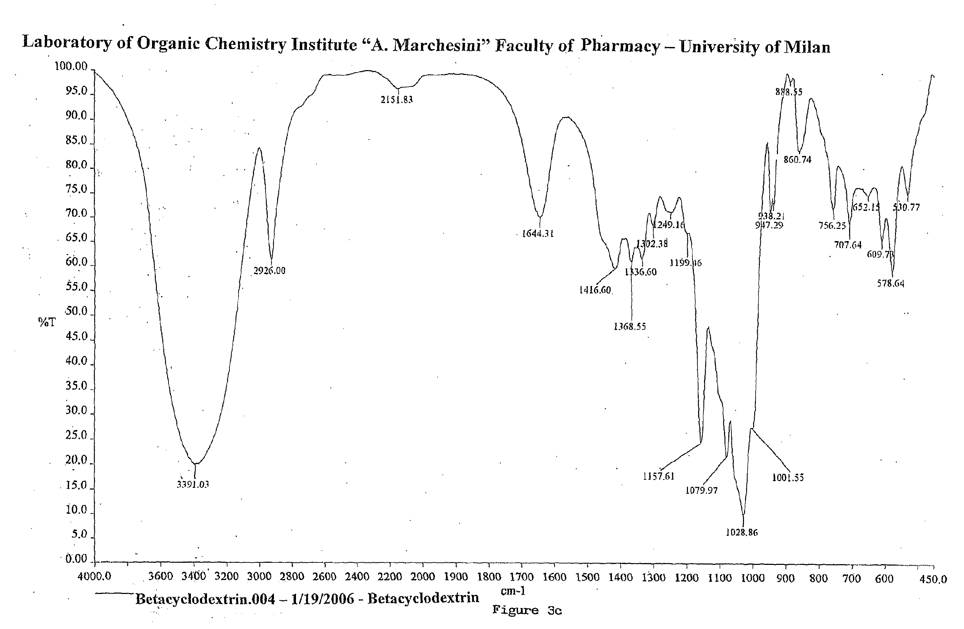

The infrared spectrum was also obtained under the same experimental conditions for a sample of raloxifene hydrochloride alone and β-cyclodextrin alone. The three obtained graphs could be compared according to Figures 3a, 3b, 3c.

As it is evident from the figures, the infrared spectrum of the complex (3a) was not the overlapping of the two separate compounds spectra. Indeed, there were characteristic bands both of raloxifene hydrochloride (3b) and β-cyclodextrin (3c), but the absorption frequency values do not overlap due to the interactions produced by the inclusion of raloxifene hydrochloride into β-cyclodextrin.

### Example 6

### Solubility test of the complex of the invention

The product as obtained from example 1 was tested for its water solubility (37°C for 48 hours), both as it is and in its micronized product form, as compared to the corresponding non-complexed starting material (raloxifene hydrochloride) in its bulk and micronized form. The results were as in the following Table 4.

**Table 4: Concentrations of raloxifene in a saturated solution under stirring at 37°C for 48 hours (200 mg of sample weighed in a suspension with 20 ml of water)**

| SAMPLE | SOLUBILITY (mg of raloxifene) |
|---|---|
| raloxifene hydrochloride | 538 |
| micronized raloxifene hydrochloride | 611 |
| raloxifene hydrochloride-β-cyclodextrin | 2417 |
| micronized raloxifene hydrochloride-β-cyclodextrin | 2848 |

It is evident that the sample of the invention, both in the form of bulk and in micronized form, had a better solubility than non-complexed raloxifene hydrochloride.

## Claims

1. An inclusion complex of raloxifene hydrochloride and β-cyclodextrin.

2. The inclusion complex according to claim 1, wherein the ratio of raloxifene hydrochloride and β-cyclodextrin is 1:1.

3. The inclusion complex according to claim 1 or claim 2, having the following peaks at diffraction degrees (2-theta) in the X-ray powder diffraction spectrum ±0.2:
10.6, 12.4, 14.4, 15.3, 19.0, 19.5.

4. The inclusion complex according to any one of the claims from 1 to 3, which has 57 peaks at the following degrees of diffraction and relative intensity:
| Peak | 2-theta | Intensity (cps) | I/I₀ |
|---|---|---|---|
| 1 | 4.4 | 370 | 15 |
| 2 | 6.2 | 320 | 13 |
| 3 | 6.6 | 351 | 14 |
| 114 | 8.9 | 452 | 18 |
| 5 | 9.4 | 463 | 18 |
| 6 | 10.6 | 817 | 32 |
| 7 | 11.7 | 480 | 19 |
| 8 | 12.4 | 2086 | 80 |
| 9 | 13.3 | 524 | 21 |
| 10 | 14.4 | 1284 | 50 |
| 11 | 15.3 | 1095 | 42 |
| 12 | 15.7 | 891 | 35 |
| 13 | 16.0 | 798 | 31 |
| 14 | 17.0 | 1016 | 39 |
| 15 | 17.7 | 1017 | 39 |
| 16 | 18.0 | 1039 | 40 |
| 17 | 19.0 | 1571 | 61 |
| 18 | 19.5 | 1602 | 62 |
| 19 | 21.1 | 2364 | 91 |
| 20 | 22.6 | 2615 | 100 |
| 21 | 24.0 | 1405 | 54 |
| 22 | 24.8 | 1049 | 41 |
| 23 | 25.0 | 1195 | 46 |
| 24 | 25.6 | 1237 | 48 |
| 25 | 26.8 | 1276 | 49 |
| 26 | 27.1 | 1378 | 53 |
| 27 | 27.5 | 1095 | 42 |
| 28 | 28.6 | 1063 | 41 |
| 29 | 29.0 | 1009 | 39 |
| 30 | 30.0 | 900 | 35 |
| 31 | 30.3 | 874 | 34 |
| 32 | 31.2 | 1122 | 43 |
| 33 | 31.8 | 1146 | 44 |
| 34 | 32.0 | 981 | 38 |
| 35 | 32.5 | 807 | 31 |
| 36 | 33.1 | 766 | 30 |
| 37 | 33.5 | 851 | 33 |
| 38 | 34.6 | 1248 | 48 |
| 39 | 35.6 | 1104 | 43 |
| 40 | 36.0 | 1007 | 39 |
| 41 | 36.5 | 1090 | 42 |
| 42 | 37.3 | 984 | 38 |
| 43 | 37.6 | 946 | 37 |
| 44 | 39.5 | 1142 | 44 |
| 45 | 39.9 | 1193 | 46 |
| 46 | 40.7 | 944 | 37 |
| 47 | 41.4 | 798 | 31 |
| 48 | 42.4 | 817 | 32 |
| 49 | 43.3 | 911 | 35 |
| 50 | 43.7 | 971 | 38 |
| 51 | 44.9 | 946 | 37 |
| 52 | 45.2 | 844 | 33 |
| 53 | 46.6 | 887 | 34 |
| 54 | 47.5 | 794 | 31 |
| 55 | 47.8 | 795 | 31 |
| 56 | 51.5 | 727 | 28 |
| 57 | 53.6 | 704 | 27 |

5. The inclusion complex according to any one of the claims from 1 to 4, wherein the complex has the diffractogram as shown in Figure 1.

6. A process for obtaining the inclusion complex of raloxifene hydrochloride with β-cyclodextrin according to any one of the claims from 1 to 5, comprising the following steps:
A) dissolving raloxifene hydrochloride in an aqueous solution;
B) heating the mass to a temperature ranging from 50 to 80°C;
C) adding β-cyclodextrin;
D) separating the raloxifene hydrochloride-β-cyclodextrin complex by precipitation and filtration.

7. The process according to claim 6, wherein the aqueous solution is a water-methanol solution.

8. An inclusion complex according to any one of the claims from 1 to 5, for the use as a medicament.

9. A pharmaceutical composition comprising the inclusion complex according to any one of the claims from 1 to 5, and a pharmaceutically acceptable vehicle.

10. A use of an inclusion complex according to any one of the claims from 1 to 5 for manufacturing a medicament for the treatment of osteoporosis.

11. The use according to claim 10, wherein the treatment is the treatment of postmenopausal osteoporosis.
